# EUROPEAN PATENT SPECIFICATION

(11) **EP 1 326 639 B1**
(45) Date of publication and mention of the grant of the patent: **16.01.2008**
(21) Application number: 01987673.9
(22) Date of filing: 16.10.2001
(51) Int. Cl.: A61K 39/39, A61K 31/335

(54) **ADJUVANT COMPOSITION COMPRISING AN IMMUNOSTIMULATORY OLIGONUCLEOTIDE AND A TOCOL**
EIN IMMUNOSTIMULIERENDES NUKLEOTID UND EIN TOCOL ENTHALTENDE ZUSAMMENSETZUNG
COMPOSITION ADJUVANTE COMPRENANT UN OLIGONUCLEOTIDE IMMUNOSTIMULATEUR ET UN TOCOL

(30) Priority: 18.10.2000 GB 0025577
(43) Date of publication of application: 16.07.2003
(73) Proprietor: GlaxoSmithKline Biologicals s.a., 1330 Rixensart (BE)
(72) Inventor: GARCON, Nathalie, B-1330 Rixensart (BE); GERARD, Catherine, Marie, Ghislaine, B-1330 Rixensart (BE); STEPHENNE, Jean, B-1330 Rixensart (BE)
(74) Representative: Easeman, Richard Lewis
(86) International application number: PCT/EP2001/011985
(87) International publication number: WO 2002/032454

(56) References cited:
- EP-A- 0 382 271
- WO-A-95/17210
- FRANCHINI A ET AL: "Vitamin E in viral inactivated vaccines." POULTRY SCIENCE, (1995 APR) 74 (4) 666-71. , XP001055520
- MUNEER R ET AL: "Evaluation of three oil- adjuvant vaccines against Pasteurella multocida in buffalo calves." REVUE SCIENTIFIQUE ET TECHNIQUE, (1994 SEP) 13 (3) 837-43. , XP001055148
- MCCLUSKIE M J ET AL: "CpG DNA is a potent enhancer of systemic and mucosal immune responses against hepatitis B surface antigen with intranasal administration to mice." JOURNAL OF IMMUNOLOGY, (1998 NOV 1) 161 (9) 4463-6. , XP002125530
- DAVIS H L ET AL: "CpG DNA is a potent enhancer of specific immunity in mice immunized with recombinant hepatitis B surface antigen." JOURNAL OF IMMUNOLOGY, (1998 JAN 15) 160 (2) 870-6. , XP002109526

## Description

The present invention relates to novel adjuvant compositions for use in vaccines. In particular, the adjuvant compositions of the present invention comprise a combination of an immunostimulatory oligonucleotide and a tocol. Also provided by the present invention are vaccines comprising the adjuvant compositions of the present invention and at least one antigen. Further provided are methods of manufacture of the adjuvant compositions and vaccines of the present invention and their use as medicaments. Additionally, the present invention provides the use of an immunostimulatory oligodeoxynucleotide and a tocol containing oil in water emulsion in the manufacture of a medicament for shifting the quality of an immune response against an antigen towards a Th1-type immune response.

Immunostimulatory oligonucleotides containing unmethylated CpG dinucleotides ("CpG") and are known adjuvants when administered by both systemic and mucosal routes (WO 96/02555, EP 468520, Davis et al., J. Immunol, 1998, 160(2):870-876; McCluskie and Davis, J. Immunol., 1998, 161(9):4463-6). CpG is an abbreviation for cytosine-guanosine dinucleotide motifs present in DNA. Historically, it was observed that the DNA fraction of BCG could exert an anti-tumour effect. In further studies, synthetic oligonucleotides derived from BCG gene sequences were shown to be capable of inducing immunostimulatory effects (both in vitro and in vivo). The authors of these studies concluded that certain palindromic sequences, including a central CG motif, carried this activity. The central role of the CG motif in immunostimulation was later elucidated in a publication by Krieg, Nature 374, p546 1995. Detailed analysis has shown that the CG motif has to be in a certain sequence context, and that such sequences are common in bacterial DNA but are rare in vertebrate DNA. The immunostimulatory sequence is often: Purine, Purine, C, G, pyrimidine, pyrimidine; wherein the dinucleotide CG motif is not methylated, but other unmethylated CpG sequences are known to be immunostimulatory and may be used in the present invention.

In certain combinations of the six nucleotides a palindromic sequence is present. Several of these motifs, either as repeats of one motif or a combination of different motifs, can be present in the same oligonucleotide. The presence of one or more of these immunostimulatory sequence containing oligonucleotides can activate various immune subsets, including natural killer cells (which produce interferon γ and have cytolytic activity) and macrophages (Wooldrige et al Vol 89 (no. 8), 1977). Although other unmethylated CpG containing sequences not having this consensus sequence have now been shown to be immunomodulatory.

CpG when formulated into vaccines, is generally administered in free solution together with free antigen (WO 96/02555; McCluskie and Davis, *supra)* or covalently conjugated to an antigen (PCT Publication No. WO 98/16247), or formulated with a carrier such as aluminium hydroxide ((Hepatitis surface antigen) Davis *et al. supra:* Brazolot-Millan et al., Proc.Natl.Acad.Sci., USA, 1998, 95(26), 15553-8). Other CpG containing formulations include those described in WO 99/61056 and WO 00/09159.

Tocols (vitamin E) and emulsion adjuvants comprising them are described in EP 0 382 271 B1 and US5667784.

Oil emulsion adjuvants have been known for many years, including work on Freunds complete and incomplete mineral oil emulsion adjuvants. Since that time much work has been performed to design stable and well tolerated alternatives to these potent, but reactogenic, adjuvant formulations. Oil emulsions comprising tocols are described in WO 95/17210.

The present invention is based on the surprising finding that combinations of immunostimulatory oligonucleotides and tocols form extremely potent adjuvant formulations. The components of these adjuvant formulations preferably interact synergistically in the induction of antigen specific antibody and are potent in the induction of immune responses conventionally associated with the Th1-type immune system. Accordingly, the adjuvant combinations are not only suitable for immunoprophylaxis of diseases, but also surprisingly for immunotherapy of diseases such as persistent viral, bacterial or parasitic infections, and also chronic disorders such as cancer.

The immunostimulatory sequence is often: Purine, Purine, C, G, pyrimidine, pyrimidine; wherein the dinucleotide CG motif is not methylated. The preferred oligonucleotides for use in adjuvants or vaccines of the present invention preferably contain two or more dinucleotide CpG motifs separated by at least three, more preferably at least six or more nucleotides. The oligonucleotides of the present invention are typically deoxynucleotides. In a preferred embodiment the internucleotide in the oligonucleotide is phosphorodithioate, or more preferably a phosphorothioate bond, although phosphodiester and other internucleotide bonds are within the scope of the invention including oligonucleotides with mixed internucleotide linkages. Methods for producing phosphorothioate oligonucleotides or phosphorodithioate are described in US5,666, 153, US5,278,302 and WO95/26204.

Examples of preferred oligonucleotides have the following sequences. The sequences preferably contain phosphorothioate modified internucleotide linkages. OLIGO 1 (SEQ ID NO:1): TCC ATG ACG TTC CTG ACG TT (CpG 1826) OLIGO 2 (SEQ ID NO:2): TCT CCC AGC GTG CGC CAT (CpG 1758) OLIGO 3 (SEQ ID NO:3): ACC GAT GAC GTC GCC GGT GAC GGC ACC ACG OLIGO 4 (SEQ ID NO:4): TCG TCG TTT TGT CGT TTT GTC GTT (CpG 2006) OLIGO 5 (SEQ ID NO:5): TCC ATG ACG TTC CTG ATG CT (CpG 1668)

Alternative CpG oligonucleotides may comprise the preferred sequences above in that they have inconsequential deletions or additions thereto.
The CpG oligonucleotides utilised in the present invention may be synthesised by any method known in the art (e.g. EP 468520). Conveniently, such oligonucleotides may be synthesised utilising an automated synthesiser.

The oligonucleotides utilised in the present invention are typically deoxynucleotides. In a preferred embodiment the internucleotide bond in the oligonucleotide is phosphorodithioate, or more preferably phosphorothioate bond, although phosphodiesters are within the scope of the present invention. Oligonucleotide comprising different internucleotide linkages are contemplated, e.g. mixed phosphorothioate phosphodiesters. Other internucleotide bonds which stabilise the oligonucleotide may be used.

The tocols that may advantageously be used in the adjuvant combinations of the present invention are described in EP 0 382 271 B1. The tocols disclosed therein are described by the general formula: 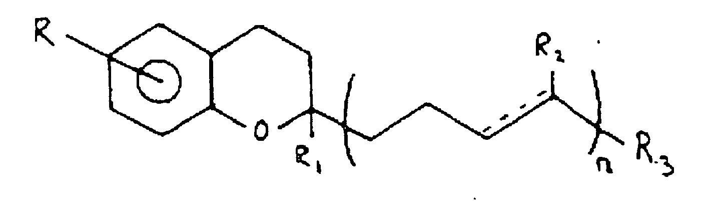 wherein R may be H or one or more identical or different substituents chosen from the group comprising alkyl, alkoxy, acyloxy, hydroxy, a sulphate and a phosphate groups; R1 and R3 independently of one another are H or alkyl; R2 is H or alkyl and may be different in each unit; the broken line indicates the presence or absence of an additional carbon-carbon bond in a unit; and n= the value 1 to 10. The alkyl group in R, R1, R2 and R3 may be chosen in particular from a linear or branched carbon chain having 1-4 carbon atoms , such as methyl, ethyl, butyl or isobutyl.

The further defined preferred subgroups are described in EP 0 382 271 B1. A particularly preferred tocol is D, L, α-tocopherol (CAS No. 10 191-4 1 -0, chemical name: (2RS,4'RS, 8'RS)-2, 5, 7, 8-tetramethyl-2-(4'. 8', 12'-trimethyl-tridecyl)-6-chromanol)); which is commercially available from ROCHE™.

Preferably the tocol is in the form of an oil emulsion, and more preferably an oil in water emulsion. The tocols may be formulated as described in EP 0 382 271 B1. in that the tocols may be dispersions of tocol droplets, optionally comprising an emulsifier, of preferably less than 1 micron in diameter. Alternatively, the tocols may be used in combination with another oil, to form the oil phase of an oil emulsion. Examples of oil emulsions which may be used in combination with the tocol are described below.

Oil in water emulsion adjuvants *per se* have been suggested to be useful as adjuvant compositions (EP 0 399 843B), also combinations of oil in water emulsions and other active agents have been described as adjuvants for vaccines (WO 95/17210; WO 98/56414; WO 99/12565; WO 99/11241). Other oil emulsion adjuvants have been described, such as water in oil emulsions (US 5,422,109; EP 0 480 982 B2) and water in oil in water emulsions (US 5,424,067; EP 0 480 981 B). All of which form preferred oil emulsion systems to incorporate tocols, and ultimately to be combined with CpG, to form adjuvants of the present invention.

The choice of the oil, for combination with the tocol, may be natural or synthetic, and may be mineral or organic. Examples of mineral and organic oils will be readily apparent to the man skilled in the art.

The oil phase of the emulsion system preferably comprises a metabolisable oil. The meaning of the term metabolisable oil is well known in the art. Metabolisable can be defined as "being capable of being transformed by metabolism" (Dorland's Illustrated Medical Dictionary, W.B. Sanders Company, 25th edition (1974)). The oil may be any vegetable oil, fish oil, animal oil or synthetic oil, which is not toxic to the recipient and is capable of being transformed by metabolism. Nuts (such as peanut oil), seeds, and grains are common sources of vegetable oils. Synthetic oils are also part of this invention and can include commercially available oils such as NEOBEE® and others. Squalene (2,6,10,15,19,23-Hexamethyl-2,6,10,14,18,22-tetracosahexaene) is an unsaturated oil which is found in large quantities in shark-liver oil, and in lower quantities in olive oil, wheat germ oil, rice bran oil, and yeast, and is a particularly preferred oil for use in this invention. Squalene is a metabolisable oil virtue of the fact that it is an intermediate in the biosynthesis of cholesterol (Merck index, 10th Edition, entry no.8619).

Particularly preferred oil emulsions for combination with CpG to form adjuvants of the present invention are oil in water emulsions, and in particular squalene in water emulsions, which comprise a tocol and an emulsifier.

Examples of preferred emulsion systems are described in WO 95/17210 and WO 99/11241 which disclose emulsion adjuvants based on squalene, α-tocopherol, and TWEEN 80, optionally formulated with the immunostimulants QS21 and/or 3D-MPL.

The size of the oil droplets found within the stable oil in water emulsion are preferably less than 1 micron, may be in the range of substantially 30-600nm, preferably substantially around 30-500nm in diameter, and most preferably substantially 150-500nm in diameter, and in particular about 150 nm in diameter as measured by photon correlation spectroscopy. In this regard, 80% of the oil droplets by number should be within the preferred ranges, more preferably more than 90% and most preferably more than 95% of the oil droplets by number are within the defined size ranges. The amounts of the components present in the oil emulsions of the present invention are conventionally in the range of from 2 to 10% oil, such as squalene; and when present, from 2 to 10% alpha tocopherol; and from 0.3 to 3% surfactant, such as polyoxyethylene sorbitan monooleate. Preferably the ratio of oil (preferably squalene): tocol (preferably α-tocopherol) is equal or less than 1 as this provides a more stable emulsion. An emulsifier, such as Tween80 or Span 85 may also be present at a level of about 1%. In some cases it may be advantageous that the vaccines of the present invention will further contain a stabiliser.

The method of producing oil in water emulsions is well known to the man skilled in the art. Commonly, the method comprises the mixing the tocol-containing oil phase with a surfactant such as a PBS/TWEEN80™ solution, followed by homogenisation using a homogenizer, it would be clear to a man skilled in the art that a method comprising passing the mixture twice through a syringe needle would be suitable for homogenising small volumes of liquid. Equally, the emulsification process in microfluidiser (M110S microfluidics machine, maximum of 50 passes, for a period of 2 minutes at maximum pressure input of 6 bar (output pressure of about 850 bar)) could be adapted by the man skilled in the art to produce smaller or larger volumes of emulsion. This adaptation could be achieved by routine experimentation comprising the measurement of the resultant emulsion until a preparation was achieved with oil droplets of the required diameter.

In an alternative embodiment of the present invention, the tocol and CpG combination may additionally comprise further immunostimulants, such as LPS or derivatives thereof, or saponins. Examples of further immunostimulants are described in "Vaccine Design-The Subunit and Adjuvant Approach" 1995, Pharmaceutical Biotechnology, Volume 6, Eds. Powell, M.F., and Newman, M.J., Plenum Press, New York and London, ISBN 0-306-44867-X.

Accordingly, the CpG/tocol adjuvant combinations of the present invention may advantageously include at least one enterobacterial lipopolysaccharide derived adjuvant. It has long been known that enterobacterial lipopolysaccharide (LPS) is a potent stimulator of the immune system, although its use in adjuvants has been curtailed by its toxic effects. A non-toxic derivative of LPS, monophosphoryl lipid A (MPL), produced by removal of the core carbohydrate group and the phosphate from the reducing-end glucosamine, has been described by Ribi et al (1986, Immunology and Immunopharmacology of bacterial endotoxins, Plenum Publ. Corp., NY, p407-419) and has the following structure:

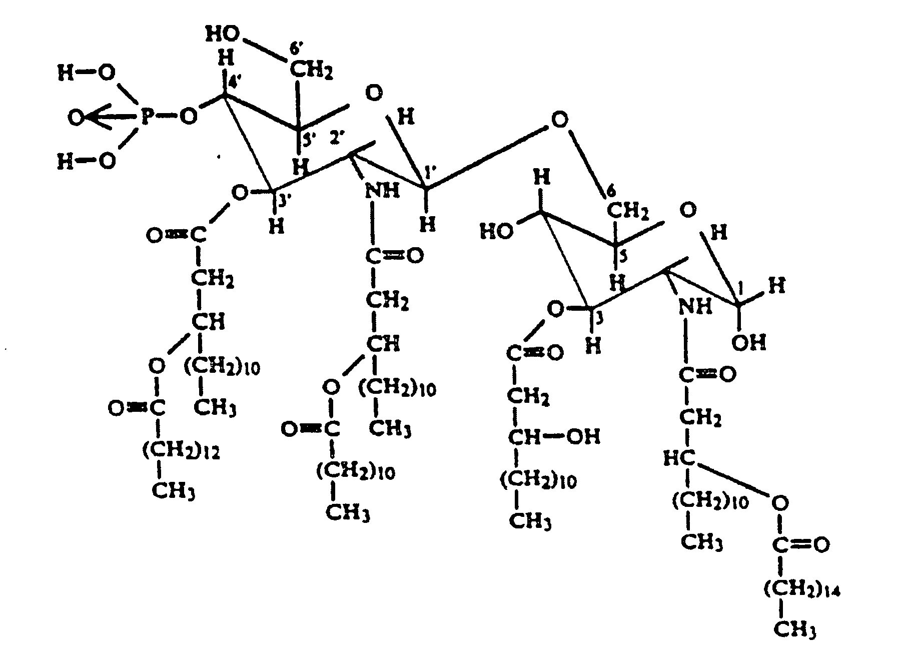

A further detoxified version of MPL results from the removal of the acyl chain from the 3-position of the disaccharide backbone, and is called 3-O-Deacylated monophosphoryl lipid A (3D-MPL). It can be purified and prepared by the methods taught in GB 2122204B, which reference also discloses the preparation of diphosphoryl lipid A, and 3-O-deacylated variants thereof. A preferred form of 3D-MPL is in the form of an emulsion having a small particle size less than 0.2µm in diameter, and its method of manufacture is disclosed in WO 94/21292. Aqueous formulations comprising monophosphoryl lipid A and a surfactant have been described in WO9843670A2.

The bacterial lipopolysaccharide derived adjuvants to be formulated in the adjuvant combinations of the present invention may be purified and processed from bacterial sources, or alternatively they may be synthetic. For example, purified monophosphoryl lipid A is described in Ribi et al 1986 (supra), and 3-O-Deacylated monophosphoryl or diphosphoryl lipid A derived from *Salmonella sp.* is described in GB 2220211 and US 4912094. Other purified and synthetic lipopolysaccharides have been described (WO 98/01139; US 6,005,099 and EP 0 729 473 B1; Hilgers et al., 1986, Int. Arch. Allergy. Immunol., 79(4):392-6; Hilgers et al., 1987, Immunology, 60(1): 141-6; and EP 0 549 074 B1). Particularly preferred bacterial lipopolysaccharide adjuvants are 3D-MPL and the β(1-6) glucosamine disaccharides described in US 6,005,099 and EP 0 729 473 B1.

Accordingly, the LPS derivatives that may be used in the present invention are those immunostimulants that are similar in structure to that of LPS or MPL or 3D-MPL. In another aspect of the present invention the LPS derivatives may be an acylated monosaccharide, which is a sub-portion to the above structure of MPL.

A preferred disaccharide adjuvant is a purified or synthetic lipid A of the following formula: 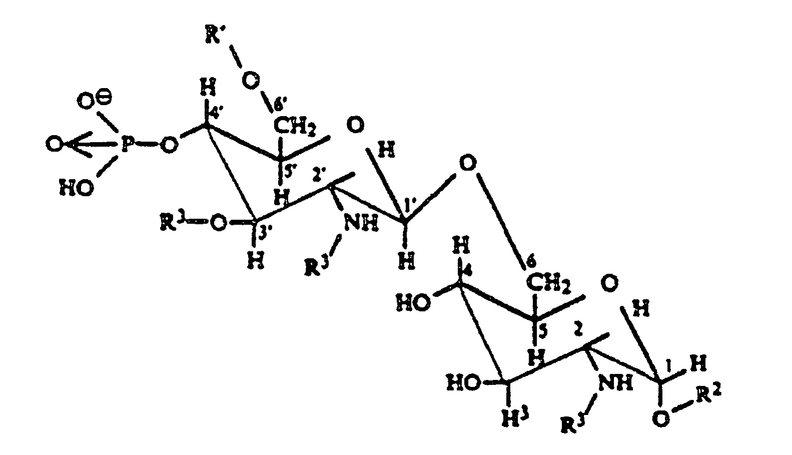 wherein R2 may be H or PO3H2: R3 may be an acyl chain or β-hydroxymyristoyl or a 3-acyloxyacyl residue having the formula: 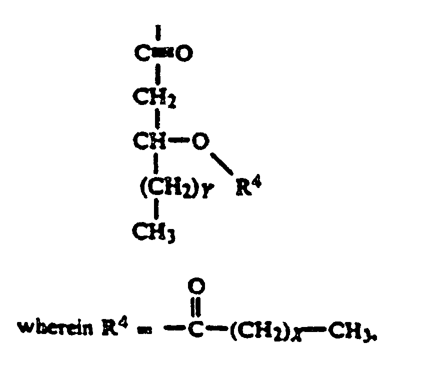 and wherein X and Y have a value of from 0 up to about 20.

The adjuvant compositions may also further comprise a saponin. Combinations of 3D-MPL and saponin adjuvants derived from the bark of *Quillaja Saponaria molina* have been described in EP 0 761 231 B. WO 95/17210 discloses an adjuvant emulsion system based on squalene, α-tocopherol, and polyoxyethylene sorbitan monooleate (TWEEN80), formulated with the immunostimulant QS21, optionally with 3D-MPL. Combinations of CpG and the adjuvant systems described in WO 95/17210 form a preferred aspect of the present invention. WO 96/33739 and WO 98/15287 describe adjuvant formulations comprising saponins and particulate carrier structures, optionally comprising 3D-MPL and/or oil in water emulsions; which when combined with a tocol and CpG form preferred adjuvants of the present invention.

Saponins are known as adjuvants in vaccines for systemic administration. The adjuvant and haemolytic activity of individual saponins has been extensively studied in the art (Lacaille-Dubois and Wagner, *supra*)*.* For example, Quil A (derived from the bark of the South American tree Quillaja Saponaria Molina), and fractions thereof, are described in US 5,057,540 and "Saponins as vaccine adjuvants", Kensil, C. R., Crit Rev Ther Drug Carrier Syst, 1996, 12 (1-2):1-55; and EP 0 362 279 B1.

Particulate structures, termed Immune Stimulating Complexes (ISCOMS), comprising fractions of Quil A are haemolytic and have been used in the manufacture of vaccines (Morein, B., EP 0 109 942 B1). These structures have been reported to have adjuvant activity (EP 0 109 942 B1; WO 96/11711).

The haemolytic saponins QS21 and QS17 (HPLC purified fractions of Quil A) have been described as potent systemic adjuvants, and the method of their production is disclosed in US Patent No.5,057,540 and EP 0 362 279 B1. Also described in these references is the use of QS7 (a non-haemolytic fraction of Quil-A) which acts as a potent adjuvant for systemic vaccines. Use of QS21 is further described in Kensil et al. (1991. J. Immunology vol 146, 431-437). Combinations of QS21 and polysorbate or cyclodextrin are also known (WO 99/10008). Particulate adjuvant systems comprising fractions of QuilA, such as QS21 and QS7 are described in WO 96/33739 and WO 96/11711. Other saponins which have been used in systemic vaccination studies include those derived from other plant species such as Gypsophila and Saponaria (Bomford et al., Vaccine, 10(9):572-577, 1992).

The adjuvant combinations of the present invention may optionally further comprise an additional carrier, such that the CpG may be associated with a particulate carrier entity to enhance the adjuvanticity of the combination. The CpG used in the adjuvant combinations of the present invention may, therefore, be in free solution or may be complexed to particulate carriers such as mineral salts (for example, but not restricted to, aluminium or calcium salts), liposomes, ISCOMs, polymers (such as, but not restricted to polylactic, polyglycolic, polyphosphazine, polyaminoacid, alginate, chitosan) or microparticles. Preferably said carriers are cationic. The CpG may also be formulated in such as way as it associates with the tocol, or tocol containing oil emulsion. This can be achieved by formulating the emulsion in such as way that it is cationic. Alternatively the CpG may be conjugated to a lipid that associates with the oil phase of the emulsion (WO 00/15256).

The vaccines of the present invention further comprise an antigen which may, or may not, be associated with the CpG-carrier complex, such as the CpG/tocol/oil droplet or the CpG/metal salt complex. The antigen may be free suspension or associated with a separate carrier. In a preferred aspect of the present invention the antigen is associated with the CpG/carrier complex.

A preferred adjuvant combination according to the present invention is composed of one or more CpG oligonucleotides containing at least 3, preferably at least 6 nucleotides between two adjacent CG motifs, together with a tocol such as D, L α-tocopherol. This combination preferably further comprises an adjuvant active derivative of LPS and a particulate carrier selected from the group comprising a metallic salt or an oil-in-water emulsion. Most preferably, the adjuvant combination comprises CpG 2006 (SEQ ID NO: 4), or CpG 1758 (SEQ ID NO: 2) or CpG 1826 (SEQ ID NO: 1) and a squalene/D, L α-tocopherol emulsion; this preferred adjuvant optionally further comprising 3-O-deacylated mono or diphosphoryl lipid A and/or QS21, or both. A preferred adjuvant system comprises the combination of QS21, 3D-MPL and the tocol and CpG.

The adjuvant combinations of the present invention may be used as both systemic or mucosal adjuvant. Vaccination of patients has been performed by many routes of administration, the most common of which is administration of the vaccine into the deep muscle of the individual (intramuscular injection). Other well known routes of vaccination include sub-cutaneous, intranasal, oral, rectal and intraperitoneal and intradermal administration. Although the vaccines of the present invention may be administered by any route, administration of the described vaccines into the skin forms a preferred embodiment of the present invention.

Human skin comprises an outer "horny" cuticle, called the stratum corneum, which overlays the epidermis. Underneath this epidermis is a layer called the dermis, which in turn overlays the subcutaneous tissue. Researchers have shown that injection of a vaccine into the skin, and in particular the dermis, stimulates an immune response, which may also be associated with a number of additional advantages. Intradermal vaccination with the vaccines described herein forms a preferred feature of the present invention.

The conventional technique of intradermal injection, the "mantoux procedure", comprises steps of cleaning the skin, and then stretching with one hand, and with the bevel of a narrow gauge needle (26-31 gauge) facing upwards the needle is inserted at an angle of between 10-15°. Once the bevel of the needle is inserted, the barrel of the needle is lowered and further advanced whilst providing a slight pressure to elevate it under the skin. The liquid is then injected very slowly thereby forming, a bleb or bump on the skin surface, followed by slow withdrawal of the needle.

More recently, devices that are specifically designed to administer liquid agents into or across the skin have been described, for example the devices described in WO 99/34850 and EP 1092444, also the jet injection devices described for example in WO 01/13977; US 5,480,381, US 5,599,302, US 5,334,144, US 5,993,412, US 5,649,912, US 5,569,189, US 5,704,911, US 5,383,851, US 5,893,397, US 5,466,220, US 5,339,163, US 5,312,335, US 5,503,627, US 5,064,413, US 5,520, 639, US 4,596,556, US 4,790,824, US 4,941,880, US 4,940,460, WO 97/37705 and WO 97/13537. Alternative methods of intradermal administration of the vaccine preparations may include conventional syringes and needles, or devices designed for ballistic delivery of solid vaccines (WO 99/27961), or transdermal patches (WO 97/48440; WO 98/28037); or applied to the surface of the skin (transdermal or transcutaneous delivery WO 98/20734 ; WO 98/28037).

When the vaccines of the present invention are to be administered to the skin, or more specifically into the dermis, the vaccine is in a low liquid volume, particularly a volume of between about 0.05 ml and 0.2 ml.

As used herein, the term "intradermal delivery" means delivery of the vaccine to the region of the dermis in the skin. However, the vaccine will not necessarily be located exclusively in the dermis. The dermis is the layer in the skin located between about 1.0 and about 2.0 mm from the surface in human skin, but there is a certain amount of variation between individuals and in different parts of the body. In general, it can be expected to reach the dermis by going 1.5 mm below the surface of the skin. The dermis is located between the stratum corneum and the epidermis at the surface and the subcutaneous layer below. Depending on the mode of delivery, the vaccine may ultimately be located solely or primarily within the dermis, or it may ultimately be distributed within the epidermis and the dermis.

The development of suitable dosing and treatment regimens for using the particular compositions described herein in a variety of treatment regimens, including *e.g.,* oral, parenteral, intravenous, intradermal, transdermal, intranasal, and intramuscular administration and formulation, is well known in the art, some of which are briefly discussed below for general purposes of illustration.

In certain applications, the pharmaceutical compositions disclosed herein may be delivered via oral administration to an animal. As such, these compositions may be formulated with an inert diluent or with an assimilable edible carrier, or they may be enclosed in hard- or soft-shell gelatin capsule, or they may be compressed into tablets, or they may be incorporated directly with the food of the diet.

The active compounds may even be incorporated with excipients and used in the form of ingestible tablets, buccal tables, troches, capsules, elixirs, suspensions, syrups, wafers, and the like (see, for example, Mathiowitz et al., Nature 1997 Mar 27;386(6623):410-4; Hwang et al., Crit Rev Ther Drug Carrier Syst 1998;15(3):243-84; U. S. Patent 5,641,515; U. S. Patent 5,580,579 and U. S. Patent 5,792,451). Tablets, troches, pills, capsules and the like may also contain any of a variety of additional components, for example, a binder, such as gum tragacanth, acacia, cornstarch, or gelatin; excipients, such as dicalcium phosphate; a disintegrating agent, such as corn starch, potato starch, alginic acid and the like; a lubricant, such as magnesium stearate; and a sweetening agent, such as sucrose, lactose or saccharin may be added or a flavoring agent, such as peppermint, oil of wintergreen, or cherry flavoring. When the dosage unit form is a capsule, it may contain, in addition to materials of the above type, a liquid carrier. Various other materials may be present as coatings or to otherwise modify the physical form of the dosage unit. For instance, tablets, pills, or capsules may be coated with shellac, sugar, or both. Of course, any material used in preparing any dosage unit form should be pharmaceutically pure and substantially non-toxic in the amounts employed. In addition, the active compounds may be incorporated into sustained-release preparation and formulations. Typically, these formulations will contain at least about 0.1 % of the active ingredients or more, although the percentage of the active ingredient(s) may, of course, be varied and may conveniently be between about 1 or 2% and about 60% or 70°,% or more of the weight or volume of the total formulation. Naturally, the amount of active compound(s) in each therapeutically useful composition may be prepared is such a way that a suitable dosage will be obtained in any given unit dose of the compound. Factors such as solubility, bioavailability, biological half-life, route of administration, product shelf life, as well as other pharmacological considerations will be contemplated by one skilled in the art of preparing such pharmaceutical formulations, and as such, a variety of dosages and treatment regimens may be desirable.

For oral administration the compositions of the present invention may alternatively be incorporated with one or more excipients in the form of a mouthwash, dentifrice, buccal tablet, oral spray, or sublingual orally-administered formulation. Alternatively, the active ingredient may be incorporated into an oral solution such as one containing sodium borate, glycerin and potassium bicarbonate, or dispersed in a dentifrice, or added in a therapeutically-effective amount to a composition that may include water, binders, abrasives, flavoring agents, foaming agents, and humectants. Alternatively the compositions may be fashioned into a tablet or solution form that may be placed under the tongue or otherwise dissolved in the mouth.

In certain circumstances it will be desirable to deliver the pharmaceutical compositions disclosed herein parenterally, intravenously, intramuscularly, or even intraperitoneally. Such approaches are well known to the skilled artisan, some of which are further described, for example, in U. S. Patent 5,543,158; U. S. Patent 5,641,515 and U. S. Patent 5,399,363. In certain embodiments, solutions of the active compounds as free base or pharmacologically acceptable salts may be prepared in water suitably mixed with a surfactant, such as hydroxypropylcellulose. Dispersions may also be prepared in glycerol, liquid polyethylene glycols, and mixtures thereof and in oils. Under ordinary conditions of storage and use, these preparations generally will contain a preservative to prevent the growth of microorganisms.

Illustrative pharmaceutical forms suitable for injectable use include sterile aqueous solutions or dispersions and sterile powders for the extemporaneous preparation of sterile injectable solutions or dispersions (for example, see U. S. Patent 5.466,468). In all cases the form must be sterile and must be fluid to the extent that easy syringability exists. It must be stable under the conditions of manufacture and storage and must be preserved against the contaminating action of microorganisms, such as bacteria and fungi. The carrier can be a solvent or dispersion medium containing, for example, water, ethanol, polyol (*e.g*., glycerol, propylene glycol, and liquid polyethylene glycol, and the like), suitable mixtures thereof, and/or vegetable oils. Proper fluidity may be maintained, for example, by the use of a coating, such as lecithin, by the maintenance of the required particle size in the case of dispersion and/or by the use of surfactants. The prevention of the action of microorganisms can be facilitated by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

In one embodiment, for parenteral administration in an aqueous solution, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, a sterile aqueous medium that can be employed will be known to those of skill in the art in light of the present disclosure. For example, one dosage may be dissolved in 1 ml of isotonic NaCl solution and either added to 1000 ml of hypodermoclysis fluid or injected at the proposed site of infusion. (see for example, "Remington's Pharmaceutical Sciences" 15th Edition, pages 1035-1038 and 1570-1580). Some variation in dosage will necessarily occur depending on the condition of the subject being treated. Moreover, for human administration, preparations will of course preferably meet sterility, pyrogenicity, and the general safety and purity standards as required by FDA Office of Biologics standards. The carriers can further comprise any and all solvents, dispersion media, vehicles, coatings, diluents, antibacterial and antifungal agents, isotonic and absorption delaying agents, buffers, carrier solutions, suspensions, colloids, and the like. The use of such media and agents for pharmaceutical active substances is well known in the art. Except insofar as any conventional media or agent is incompatible with the active ingredient, its use in the therapeutic compositions is contemplated. Supplementary active ingredients can also be incorporated into the compositions. The phrase "pharmaceutically-acceptable" refers to molecular entities and compositions that do not produce an allergic or similar untoward reaction when administered to a human.

In certain embodiments, the pharmaceutical compositions may be delivered by intranasal sprays, inhalation, and/or other aerosol delivery vehicles. Methods for delivering genes, nucleic acids, and peptide compositions directly to the lungs *via* nasal aerosol sprays has been described, *e.g.,* in U. S. Patent 5,756,353 and U. S. Patent 5,804.212. Likewise, the delivery of drugs using intranasal microparticle resins (Takenaga et al., J Controlled Release 1998 Mar 2;52 (1-2):81-7) and lysophosphatidyl-glycerol compounds (U. S. Patent 5,725,871) are also well-known in the pharmaceutical arts. Likewise, illustrative transmucosal drug delivery in the form of a polytetrafluoroetheylene support matrix is described in U. S. Patent 5,780,045.

Methods of systemic administration of the vaccine preparations may include conventional syringes and needles, or devices designed for ballistic delivery of solid vaccines (WO 99/27961), or needleless pressure liquid jet device (US 4,596,556; US 5,993,412), or transdermal patches (WO 97/48440; WO 98/28037). The present invention may also be used to enhance the immunogenicity of antigens applied to the skin (transdermal or transcutaneous delivery WO 98/20734 ; WO 98/28037). The present invention therefore provides a delivery device for systemic administration, pre-filled with the vaccine or adjuvant compositions of the present invention.

The present invention therefore provides a delivery device for systemic administration, pre-filled with the vaccine or adjuvant compositions of the present invention. Accordingly there is provided a method for inducing an immune response in an individual, comprising the administration of a vaccines of the present invention. to the individual, wherein the vaccine is administered via the parenteral or systemic route.

Preferably the vaccine formulations of the present invention contain an antigen or antigenic composition capable of eliciting an immune response against a human pathogen, which antigen or antigenic composition is derived from HIV-1. (such as tat. nef, gp120 or gp160), human herpes viruses, such as gD or derivatives thereof or Immediate Early protein such as ICP27 from HSV 1 or HSV2, cytomegalovirus ((esp Human)(such as gB or derivatives thereof), Rotavirus (including live-attenuated viruses), Epstein Barr virus (such as gp350 or derivatives thereof), Varicella Zoster Virus (such as gpI, II and IE63), or from a hepatitis virus such as hepatitis B virus (for example Hepatitis B Surface antigen or a derivative thereof), hepatitis A virus, hepatitis C virus and hepatitis E virus, or from other viral pathogens, such as paramyxoviruses: Respiratory Syncytial virus (such as F and G proteins or derivatives thereof), parainfluenza virus, measles virus, mumps virus, human papilloma viruses (for example HPV6, 11. 16, 18,..), flaviviruses (e.g. Yellow Fever Virus, Dengue Virus, Tick-borne encephalitis virus, Japanese Encephalitis Virus) or Influenza virus (whole live or inactivated virus, split influenza virus, grown in eggs or MDCK cells, or whole flu virosomes (as described by R. Gluck, Vaccine, 1992, 10, 915-920) or purified or recombinant proteins thereof, such as HA, NP, NA, or M proteins, or combinations thereof), or derived from bacterial pathogens such as *Neisseria spp,* including *N. gonorrhea* and *N. meningitidis* (for example capsular polysaccharides and conjugates thereof, transferrin-binding proteins, lactoferrin binding proteins, PilC, adhesins); *S. pyogenes* (for example M proteins or fragments thereof, C5A protease, lipoteichoic acids), *S. agalactiae. S. mutans; H. ducreyi; Moraxella spp, including M catarrhalis, also known as Branhamella* catarrhalis (for example high and low molecular weight adhesins and invasins); *Bordetella spp, including B. pertussis* (for example pertactin, pertussis toxin or derivatives thereof, filamenteous hemagglutinin, adenylate cyclase, fimbriae), *B. parapertussis and B. bronchiseptica; Mycobacterium spp., including M. tuberculosis* (for example ESAT6, Antigen 85A, -B or -C), *M. bovis, M. leprae. M. avium. M. paratuberculosis. M. smegmatis; Legionella spp,* including *L. pneumophila; Escherichia spp,* including *enterotoxic E. coli* (for example colonization factors, heat-labile toxin or derivatives thereof, heat-stable toxin or derivatives thereof), enterohemorragic *E*. *coli,* enteropathogenic *E. coli* (for example shiga toxin-like toxin or derivatives thereof); *Vibrio spp,* including *V. cholera* (for example cholera toxin or derivatives thereof); *Shigella spp,* including *S*. *sonnei, S. dysenteriae, S. flexnerii; Yersinia spp,* including *Y.* enterocolitica (for example a Yop protein), *Y*. *pestis, Y*. *pseudotuberculosis*; *Campylobacter spp,* including *C. jejuni* (for example toxins, adhesins and invasins) and *C. coli; Salmonella spp,* including S. *typhi, S. paratyphi, S. choleraesuis, S. enteritidis; Listeria spp.,* including L. *monocytogenes : Helicobacter spp,* including *H.* pylori (for example urease, catalase, vacuolating toxin); *Pseudomonas spp,* including *P. aeruginosa: Staphylococcus spp.,* including *S*. *aureus, S, epidermidis; Enterococcus spp.,* including *E. faecalis, E. faecium; Clostridium spp.*, including *C.* tetani (for example tetanus toxin and derivative thereof), C. *botulinum* (for example botulinum toxin and derivative thereof), *C. difficile* (for example clostridium toxins A or B and derivatives thereof); *Bacillus spp.,* including *B. anthracis* (for example botulinum toxin and derivatives thereof); *Corynebacterium spp.,* including C. *diphtheriae* (for example diphtheria toxin and derivatives thereof); *Borrelia spp.,* including *B. burgdorferi* (for example OspA, OspC, DbpA, DbpB), *B. garinii* (for example OspA, OspC, DbpA, DbpB), *B. afzelii* (for example OspA, OspC, DbpA, DbpB), *B. andersonii* (for example OspA, OspC, DbpA, DbpB), *B. hermsii; Ehrlichia spp.,* including *E. equi* and the agent of the Human Granulocytic Ehrlichiosis; *Rickettsia spp,* including *R. rickettsii; Chlamydia spp., including C. trachomatis* (for example MOMP, heparin-binding proteins), *C*. *pneumoniae* (for example MOMP, heparin-binding proteins), *C. psittaci; Leptospira spp.,* including *L. interrogans; Treponema spp.,* including *T. pallidum* (for example the rare outer membrane proteins), *T. denticola, T. hyodysenteriae:* or derived from parasites such as *Plasmodium spp.,* including *P. falciparum*; *Toxoplasma spp.,* including *T. gondii (for example SAG2, SAG3, Tg34); Entamoeba spp.,* including *E*. *histolytica; Babesia spp.,* including *B. microti; Trypanosoma spp.,* including *T. cruzi; Giardia spp.,* including *G*. *lamblia; Leshmania spp.,* including L. *major; Pneumocystis spp.,* including *P. carinii; Trichomonas spp.,* including *T*. *vaginalis; Schisostoma spp.,* including *S*. *mansoni,* or derived from yeast such as *Candida spp.,* including *C*. *albicans; Cryptococcus spp.,* including *C*. *neoformans.*

Other preferred specific antigens for *M. tuberculosis* are for example Tb Ra12. Tb H9, Tb Ra35. Tb38-1, Erd 14, DPV, MTI, MSL, mTTC2 and hTCC 1 (WO 99/51748). Proteins for *M. tuberculosis* also include fusion proteins and variants thereof where at least two, preferably three polypeptides of *M*. *tuberculosis* are fused into a larger protein. Preferred fusions include Ra12-TbH9-Ra35, Erd14-DPV-MTI, DPV-MTI-MSL, Erd14-DPV-MTI-MSL-mTCC2, Erd14-DPV-MTI-MSL, DPV-MTI-MSL-mTCC2, TbH9-DPV-MTI (WO 99/51748).

Most preferred antigens for Chlamydia include for example the High Molecular Weight Protein (HWMP) (WO 99/17741), ORF3 (EP 366 412), and putative membrane proteins (Pmps). Other Chlamydia antigens of the vaccine formulation can be selected from the group described in WO 99/28475.

Preferred bacterial vaccines comprise antigens derived from *Streptococcus spp,* including *S*. *pneumoniae* (for example capsular polysaccharides and conjugates thereof, PsaA, PspA, streptolysin, choline-binding proteins) and the protein antigen Pneumolysin (Biochem Biophys Acta, 1989, 67, 1007; Rubins et al., Microbial Pathogenesis, 25, 337-342), and mutant detoxified derivatives thereof (WO 90/06951; WO 99/03884). Other preferred bacterial vaccines comprise antigens derived from *Haemophilus spp., including H. influenzae type B* (for example PRP and conjugates thereof), *non typeable H. influenzae,* for example OMP26, high molecular weight adhesins, P5, P6, protein D and lipoprotein D, and fimbrin and fimbrin derived peptides (US 5,843,464) or multiple copy varients or fusion proteins thereof.

Derivatives of Hepatitis B Surface antigen are well known in the art and include, inter alia, those PreS1, PreS2 S antigens set forth described in European Patent applications EP-A-414 374; EP-A-0304 578, and EP 198-474. In one preferred aspect the vaccine formulation of the invention comprises the HIV-1 antigen, gp120, especially when expressed in CHO cells. In a further embodiment, the vaccine formulation of the invention comprises gD2t as hereinabove defined.

In a preferred embodiment of the present invention vaccines containing the claimed adjuvant comprise antigen derived from the Human Papilloma Virus (HPV) considered to be responsible for genital warts (HPV 6 or HPV 11 and others), and the HPV viruses responsible for cervical cancer (HPV16, HPV18 and others).

Particularly preferred forms of genital wart prophylactic, or therapeutic, vaccine comprise L 1 particles or capsomers, and fusion proteins comprising one or more antigens selected from the HPV 6 and HPV 11 proteins E6, E7, L1, and L2.

The most preferred forms of fusion protein are: L2E7 as disclosed in WO 96/26277. and proteinD(1/3)-E7 disclosed in GB 9717953.5 (PCT/EP98/05285).

A preferred HPV cervical infection or cancer, prophylaxis or therapeutic vaccine, composition may comprise HPV 16 or 18 antigens. For example, L1 or L2 antigen monomers, or L 1 or L2 antigens presented together as a virus like particle (VLP) or the L1 alone protein presented alone in a VLP or caposmer structure. Such antigens, virus like particles and capsomer are per se known. See for example WO94/00152, WO94/20137, WO94/05792, and WO93/02184.

Additional early proteins may be included alone or as fusion proteins such as E7. E2 or preferably E5 for example: particularly preferred embodiments of this includes a VLP comprising L1E7 fusion proteins (WO 96/11272).

Particularly preferred HPV 16 antigens comprise the early proteins E6 or E7 in fusion with a protein D carrier to form Protein D - E6 or E7 fusions from HPV 16, or combinations thereof; or combinations of E6 or E7 with L2 (WO 96/26277).

Alternatively the HPV 16 or 18 early proteins E6 and E7, may be presented in a single molecule, preferably a Protein D- E6/E7 fusion. Such vaccine may optionally contain either or both E6 and E7 proteins from HPV 18, preferably in the form of a Protein D - E6 or Protein D - E7 fusion protein or Protein D E6/E7 fusion protein.

The vaccine of the present invention may additionally comprise antigens from other HPV strains, preferably from strains HPV 31 or 33.

Vaccines of the present invention further comprise antigens derived from parasites that cause Malaria. For example, preferred antigens from *Plasmodia falciparum* include RTS.S and TRAP. RTS is a hybrid protein comprising substantially all the C-terminal portion of the circumsporozoite (CS) protein of *P.falciparum* linked via four amino acids of the preS2 portion of Hepatitis B surface antigen to the surface (S) antigen of hepatitis B virus. It's full structure is disclosed in the International Patent Application No. PCT/EP92/02591, published under Number WO 93/10152 claiming priority from UK patent application No.9124390.7. When expressed in yeast RTS is produced as a lipoprotein particle, and when it is co-expressed with the S antigen from HBV it produces a mixed particle known as RTS,S. TRAP antigens are described in the International Patent Application No. PCT/GB89/00895, published under WO 90/01496. A preferred embodiment of the present invention is a Malaria vaccine wherein the antigenic preparation comprises a combination of the RTS,S and TRAP antigens. Other plasmodia antigens that are likely candidates to be components of a multistage Malaria vaccine are *P. faciparum* MSP1, AMA1, MSP3, EBA, GLURP, RAP1, RAP2, Sequestrin, PfEMP1, Pf332, LSA1, LSA3, STARP, SALSA, PfEXP1, Pfs25, Pfs28, PFS27/25, Pfs16, Pfs48/45. Pfs230 and their analogues in Plasmodium spp.

The formulations may also contain an anti-tumour antigen and be useful for the immunotherapeutic treatment of cancers. For example, the adjuvant formulation finds utility with tumour rejection antigens such as those for prostrate, breast, colorectal, lung, pancreatic, renal or melanoma cancers. Exemplary antigens include MAGE 1 3 and MAGE 4 or other MAGE antigens such as disclosed in WO99/40188, PRAME, BAGE, Lage (also known as NY Eos 1) SAGE and HAGE (WO 99/53061) or GAGE (Robbins and Kawakami, 1996, Current Opinions in Immunology 8, pps 628-636; Van den Eynde et al., International Journal of Clinical & Laboratory Research (submitted 1997); Correale et al. (1997), Journal of the National Cancer Institute 89, p293. Indeed these antigens are expressed in a wide range of tumour types such as melanoma, lung carcinoma, sarcoma and bladder carcinoma.

MAGE antigens for use in the present invention may be expressed as a fusion protein with an expression enhancer or an Immunological fusion partner. In particular, the Mage protein may be fused to Protein D from Heamophilus infuenzae B or a lipidated derivative thereof.. In particular, the fusion partner may comprise the first 1/3 of Protein D. Such constructs are disclosed in Wo99/40188.

Other tumour-specific antigens are suitable for use with the adjuvants of the present invention and include, but are not restricted to tumour-specific gangliosides such as GM 2, and GM3 or conjugates thereof to carrier proteins; or said antigen may be a self peptide hormone such as whole length Gonadotrophin hormone releasing hormone (GnRH, WO 95/20600), a short 10 amino acid long peptide, useful in the treatment of many cancers, or in immunocastration.

In a preferred embodiment prostate antigens are utilised, such as Prostate specific antigen (PSA), PAP, PSCA (PNAS 95(4) 1735-1740 1998), PSMA or antigen known as Prostase.

Prostase is a prostate-specific serine protease (trypsin-like), 254 amino acid-long, with a conserved serine protease catalytic triad H-D-S and a amino-terminal pre-propeptide sequence, indicating a potential secretory function (P. Nelson, Lu Gan, C. Ferguson, P. Moss, R. Gelinas, L. Hood & K. Wand, "Molecular cloning and characterisation of prostase, an androgen-regulated serine protease with prostate restricted expression, In Proc. Natl. Acad. Sci. USA (1999) 96, 3114-3119). A putative glycosylation site has been described. The predicted structure is very similar to other known serine proteases, showing that the mature polypeptide folds into a single domain. The mature protein is 224 amino acids-long, with one A2 epitope shown to be naturally processed.

Prostase nucleotide sequence and deduced polypeptide sequence and homologs are disclosed in Ferguson, et al. (Proc. Natl. Acad. Sci. USA 1999, 96, 3114-3119) and in International Patent Applications No. WO 98/12302 (and also the corresponding granted patent US 5,955,306). WO 98/20117 (and also the corresponding granted patents US 5,840,871 and US 5,756,145) (prostate-specific kallikrein) and WO 00/04149 (P703P).

The present invention provides formulations comprising prostase protein fusions based on prostase protein and fragments and homologues thereof ("derivatives"). Such derivatives are suitable for use in therapeutic vaccine formulations which are suitable for the treatment of a prostate tumours. Typically the fragment will contain at least 20, preferably 50, more preferably 100 contiguous amino acids as disclosed in the above referenced patent and patent applications.

A further preferred prostate antigen is known as P501S, sequence ID no 113 of Wo98/37814. Immunogenic fragments and portions thereof comprising at least 20, preferably 50, more preferably 100 contiguous amino acids as disclosed in the above referenced patent application. See for example PS 108 (WO 98/50567).

Other prostate specific antigens are known from WO98/37418, and WO/004149. Another is STEAP PNAS 96 14523 14528 7 -12 1999.

Other tumour associated antigens useful in the context of the present invention include: Plu -1 J Biol. Chem 274 (22) 15633 -15645, 1999, HASH -1, HasH-2, Cripto (Salomon et al Bioessays 199, 21 61-70,US patent 5654140) Criptin US patent 5 981 215, ., Additionally, antigens particularly relevant for vaccines in the therapy of cancer also comprise tyrosinase and survivin.

Mucin dervied peptides such as Muc1 see for example US 5744,144 US 5827, 666 WO 8805054, US 4,963,484. Specifically contemplated are Muc 1 derived peptides that comprise at least one repeat unit of the the Muc 1 peptide, preferably at least two such repeats and which is recognised by the SM3 antibody (US 6 054 438). Other mucin derived peptides include peptide from Muc 5.

The present invention is also useful in combination with breast cancer antigens such as her 2/ Neu, mammaglobin (US patent 5668267) or those disclosed in WO/00 52165, WO99/33869, WO99/19479, WO 98/45328. Her 2 neu antigens are disclosed inter alia, in US patent 5,801,005. Preferably the Her 2 neu comprises the entire extracellular domain (comprising approximately amino acid 1 -645) or fragments thereof and at least an immunogenic portion of or the entire intracellular domain approximately the C terminal 580 amino acids. In particular, the intracellular portion should comprise the phosphorylation domain or fragments thereof. Such constructs are disclosed in WO00/44899. A particularly preferred construct is known as ECD PD a second is known as ECD PD See WO/00/44899. The her 2 neu as used herein can be derived from rat, mouse or human.

The formulations may contain antigens associated with tumour-support mechanisms (e.g. angiogenesis, tumour invasion) for example tie 2, VEGF.

It is foreseen that compositions of the present invention will be used to formulate vaccines containing antigens derived from *Borrelia sp..* For example, antigens may include nucleic acid, pathogen derived antigen or antigenic preparations, recombinantly produced protein or peptides, and chimeric fusion proteins. In particular the antigen is OspA. The OspA may be a full mature protein in a lipidated form virtue of the host cell (E.Coli) termed (Lipo-OspA) or a non-lipidated derivative. Such non-lipidated derivatives include the non-lipidated NS1-OspA fusion protein which has the first 81 N-terminal amino acids of the non-structural protein (NS1) of the influenza virus, and the complete OspA protein, and another, MDP-OspA is a non-lipidated form of OspA carrying 3 additional N-terminal amino acids.

Vaccines of the present invention may be used for the prophylaxis or therapy of allergy. Such vaccines would comprise allergen specific (for example Der p1) and allergen non-specific antigens (for example peptides derived from human IgE, including but not restricted to the stanworth decapeptide (EP 0 477 231 B1)).

Vaccines of the present invention may also be used for the prophylaxis or therapy of chronic disorders others than allergy, cancer or infectious diseases. Such chronic disorders are diseases such as atherosclerosis, and Alzheimer.

The vaccines of the present invention are particularly suited for the immunotherapeutic treatment of diseases, such as chronic conditions and cancers, but also for the therapy of persistent infections. Accordingly the vaccines of the present invention are particularly suitable for the immunotherapy of infectious diseases, such as Tuberculosis (TB), AIDS and Hepatitis B (HepB) virus infections.

Accordingly there is provided vaccines and adjuvants of the present invention for the immunotherapy of infectious diseases such as TB, AIDS and HepB; and the use of such adjuvants and vaccines in the manufacture of medicaments for the immunotherapy of infectious diseases such as TB, AIDS and HepB. In the context of TB, there is provided a method of treating an individual suffering from TB infection, comprising the administration of a vaccine of the present invention to the individual, thereby reducing the bacterial load of that individual. The reduction of bacterial load, consisting of a reduction of the amount of TB found in the lung sputum, leading to the amelioration or cure of the TB disease.

Also, in the context of AIDS, there is provided a method of treatment of an individual susceptible to or suffering from AIDS. The method comprising the administration of a vaccine of the present invention to the individual, thereby reducing the amount of CD4+ T-cell decline caused by subsequent HIV infection, or slowing or halting the CD4+ T-cell decline in an individual already infected with HIV.

Additionally, in the context of persistent Hepatitis B virus infection, there is provided a method of treatment of an individual susceptible to or suffering from HepB infection. Accordingly, there is provided a method comprising the administration of a vaccine of the present invention to the individual, thereby reducing the level of HepB load in the serum (as measured by DNA clearance) and also reducing the amount of liver damage (as detected by the reduction or stabilisation of serum levels of the enzyme Alanine Transferase (ALT)).

The amount of protein in each vaccine dose is selected as an amount which induces an immunoprotective response without significant, adverse side effects in typical vaccinees. Such amount will vary depending upon which specific immunogen is employed and how it is presented. Generally, it is expected that each dose will comprise 1-1000 µg of protein, preferably 1-500 µg, preferably 1-100µg, most preferably 1 to 50µg. An optimal amount for a particular vaccine can be ascertained by standard studies involving observation of appropriate immune responses in vaccinated subjects.

The amount of CpG or immunostimulatory oligonucleotides in the adjuvants or vaccines of the present invention is generally small, but depending on the vaccine formulation may be in the region of 1-1000µg per dose, preferably 1-500µg per dose, and more preferably between 1 to 100µg per dose.

If present, the amount of LPS derivative for use in the adjuvants of the present invention may be in the region of 1-1000µg per dose, preferably 1-500µg per dose, more preferably 1-250µg per dose, and most preferably between 1 to 100µg per dose. The ratio of CpG:LPS derivative (w/w) will, therefore, be in the range of 1:1000 to 1000:1, and will typically be in the range of 1:100 to 100:1, and preferably in the range of 1:10 to 1:1 or 1:1 to 10:1, and most preferably 1:1, 4:1 or 10:1.

The formulations of the present invention maybe used for both prophylactic and therapeutic purposes. Accordingly, there is provided the use of a combination of a saponin and a CpG molecule in the manufacture of a vaccine for the prophylaxis and the treatment of viral, bacterial, parasitic infections, allergy, cancer and other non-chronic disorders. Accordingly, the present invention provides for a method of treating a mammal susceptible to or suffering from an infectious disease or cancer, or allergy, or autoimmune disease. In a further aspect of the present invention there is provided a vaccine or adjuvant combination, comprising a saponin and CpG, as herein described for use as a medicament. Vaccine preparation is generally described in New Trends and Developments in Vaccines, edited by Voller et al., University Park Press. Baltimore, Maryland, U.S.A. 1978.

It is foreseen that compositions of the present invention will be used to formulate vaccines containing antigens derived from a wide variety of sources. For example, antigens may include human, bacterial, or viral nucleic acid, pathogen derived antigen or antigenic preparations, tumour derived antigen or antigenic preparations, host-derived antigens, including peptides derived from IgE, such as the histamine releasing decapeptide of IgE (known as the Stanworth decapeptide), recombinantly produced protein or peptides, and chimeric fusion proteins.

There is also provided by the present invention a method of shifting the quality of an immune response against an antigen, generated by a vaccine comprising an immunostimulatory oligonucleotide, towards a Th1-type immune response, the method comprising formulating the vaccine with an immunostimulatory oligonucleotide and a tocol containing oil in water emulsion. Specifically, the method of shifting the quality of an immune response results in the generation of a Th1-type immune response as assayed in a mouse model in that antigen specific IgG isotypes induced by the vaccine are characterised in that IgG1 constitutes less than 50% of the total antigen specific IgG as determined by mid point titres measured by isotype specific ELISA. Preferably less than 40% of the total antigen specific IgG as determined by mid point titres measured by isotype specific ELISA.

There also provided a method of manufacturing a vaccine composition comprising formulating an oil in water emulsion comprising a tocol, admixing said tocol emulsion with an immunostimulatory oligonucleotide to form an adjuvant, and formulating said adjuvant with an antigen or antigenic composition.

There is also provided a method of treating an individual susceptible to or suffering from a disease comprising the administration to said individual of a vaccine composition comprising a combination of an immunostimulatory oligonucleotide and a tocol. The use of the vaccines as described herein in medicine is also provided.

Further there is provided a method of manufacture of a vaccine or adjuvant are also provided, comprising taking a derivative of LPS, and taking a CpG molecule and admixing them with an antigen in a pharmaceutically acceptable excipient, in the absence of a saponin. Examples of suitable pharmaceutically acceptable excipients for use in the combinations of the present invention include water, phosphate buffered saline, isotonic buffer solutions.

Preferably the adjuvants of the present invention consists or consists essentially of a tocol and metabolisable oil emulsion and an immunostimulatory oligonucleotide. More preferably the adjuvant compositions consist essentially of a tocol and metabolisable oil emulsion, an immunostimulatory oligonucleotide and a saponin. In both of these above two embodiments the tocol is preferably α-tocopherol, the metabolisable oil is preferably squalene and the immunostimulatory oligonucleotide is preferably CpG 2006 (SEQ ID NO. 4); all variations and combinations within these embodiments are clearly envisaged by the present invention. Most preferably the adjuvant compositions consists or consists essentially of α-tocopherol and squalene emulsion, CpG 2006 (SEQ ID NO. 4 and QS21.

The most preferred vaccines of the present invention contain a Her2Neu antigen, preferably a fusion of the extracellular domain of Her2Neu linked to the phosphorylation domain (ECD-PD (produced as described in WO 00/44899)). Accordingly there is provided by the present invention a method of treating an individual suffering from cancer comprising the administration to said individual of a vaccine composition comprising a combination of an immunostimulatory oligonucleotide, a tocol, and ECD-PD. All the preferred features of the immunostimulatory oligonucleotide and tocol which have been described above are also envisaged in this method of treatment.

The present invention is exemplified, but not limited to, the following

### examples.

### Example 1, Immunisation of mice with ECD-PD antigen

This experiment was designed to investigate a range of adjuvant formulations with the antigen which is a fusion of the extracellular domain of Her2Neu linked to the phosphorylation domain (ECD-PD), which was produced in CHO cells according to the methods of WO 00/44899.

| Group | Antigen (25µg) | Adjuvant |
|---|---|---|
| 1 | ECD-PD | none (Phosphate Buffered Saline (PBS)) |
| 2 | ECD-PD | Liposomes with QS21 and 3D-MPL in membrane |
| 3 | ECD-PD | tocol containing oil in water emulsion with QS21 and 3D-MPL |
| 4 | ECD-PD | CpG |
| 5 | ECD-PD | Liposomes with QS21 and 3D-MPL in membrane + CpG |
| 6 | ECD-PD | tocol containing oil in water emulsion with QS21 and 3D-MPL + CpG |
| 7 | ECD-PD | 3D-MPL+CpG |
| 8 | ECD-PD | QS21+CpG |
| 9 | ECD-PD | tocol containing oil in water emulsion + CpG |
| 10 | ECD-PD | Liposomes with QS21 in membrane + CpG |
| 11 | ECD-PD | Liposomes with 3D-MPL in membrane + CpG |

The tocol containing oil in water emulsions used in the above groups used D, L. α-tocopherol (CAS No. 10191-41-0; chemical name: (2RS,4'RS, 8'RS)-2, 5, 7, 8-tetramethyl-2-(4', 8', 12'-trimethyl-tridecyl)-6-chromanol)); which is commercially available from ROCHE™. If present the tocol was present in an oil in water emulsion comprising 2.5% by volume, in combination with squalene 2.5% by volume. Both oils were mixed, and polyoxyethylene sorbitan monooleate (Tween 80™) was added, prior to microfluidisation (M110S microfluidics machine, maximum of 50 passes, for a period of 2 minutes at maximum pressure input of 6 bar (output pressure of about 850 bar) as described in WO 95/17210). Accordingly, groups 3,6, and 9 were based on the above tocol emulsion with the addition of aqueous QS21, 3D-MPL or CpG.

QS21 and 3D-MPL if present in any of the vaccine groups above were included at 5µg/dose; CpG (OLIGO 4 (SEQ ID NO:4): TCG TCG TTT TGT CGT TTT GTC GTT) was added at 50µg dose.

The adjuvants as used for group 2, 5, 10 were prepared according to techniques as described in EP 0 822 831 B1 Group 11 comprised 3D-MPL in the membrane of a liposome. Briefly, the 3D-MPL, dioleoyl phosphatydyl choline and cholesterol were mixed together and microfluidised into unilamellar liposomes (as described in EP 0 822 831 B 1 - with the omission of QS21).

The adjuvants used in groups 4, 7 and 8 were in aqueous suspension or solution.

### Vaccination procedure

Groups of B6F mice were vaccinated on four occasions (in 50µl volumes), intramuscularly, 14 days apart. 14 days post the 4th vaccine dose, the mice were challenged subcutaneously with 2X 10⁶ TC 1 tumour cell expressing the Her2Neu.

### Tumour cell line TC1:

Primary lung epithelial cells from C57BL.6 mice were immortalised by HPV 16 E6 and E7 and then transformed with an activated ras oncogene, producing a tumourigenic cell line expressing E6 and E7 (Lin KY Cancer Res 1996 Jan 1;56(1):21-6l*).* The E7 expression has been verified by FACS analysis of fixed and permeabilised TC1 cells using the mouse anti-HPV 16 E7 Mab (Triton Corp. Alameda, CA). The Her2Neu-TC 1 tumour cell lines was produced by transduction of these TC 1 cells by retroviral vectors coding for Her2Neu. After a selection period with blastocydin, resistant clones were isolated and screened by FACS for Her2Neu expression. The clone with the highest Her2Neu expression was selected, and a challenge dose of 2X10⁶ cells was identified to have a similar Kinetic of growth as the wild-type TC 1 cells and to give rise to a developing tumour in 100% of the control animals.

The size of the individual tumors were measured twice a week and expressed as a group mean.

### Results

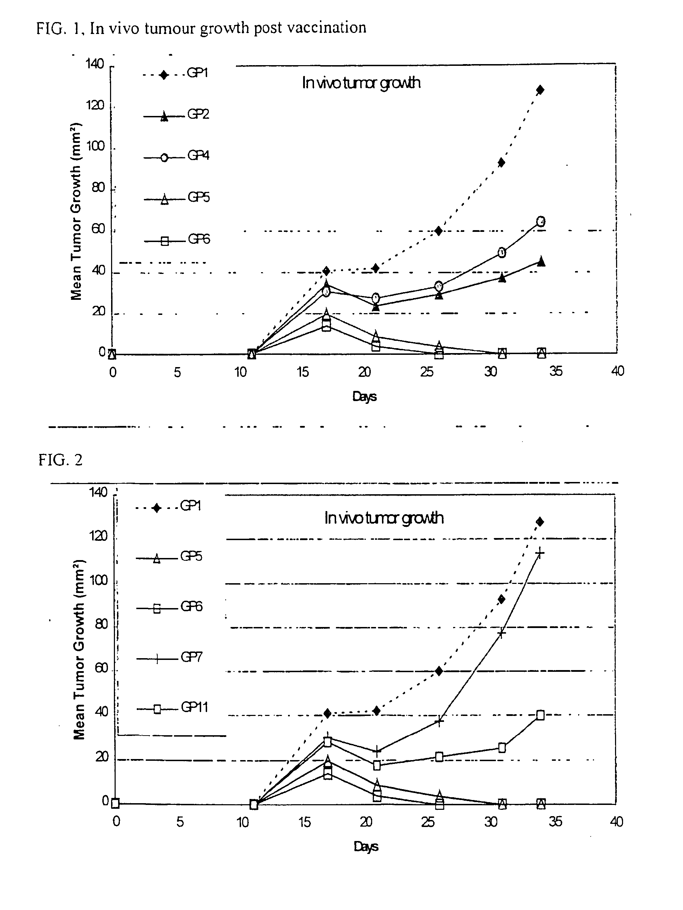

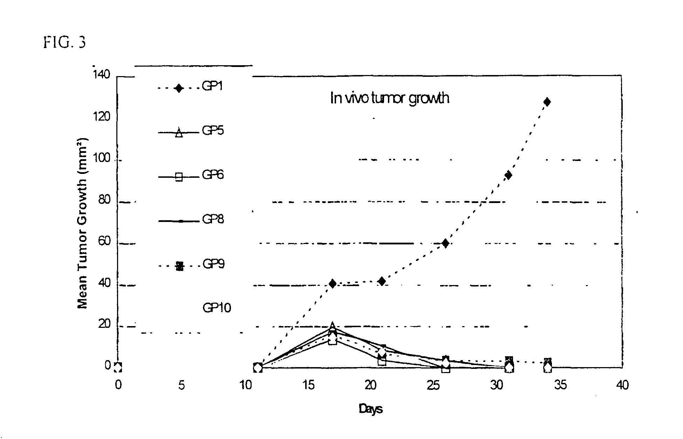

Figure 1 shows the tumour growth results for groups 1, 2, 4, 5 and 6. Figure 2 shows the tumour growth results for groups 1, 5, 6, 7 and 11. Figure 3 shows the tumour growth results for groups 1, 5, 6, 8, 9 and 10. Formulations comprising a tocol and CpG induced a complete regression of the tumor.

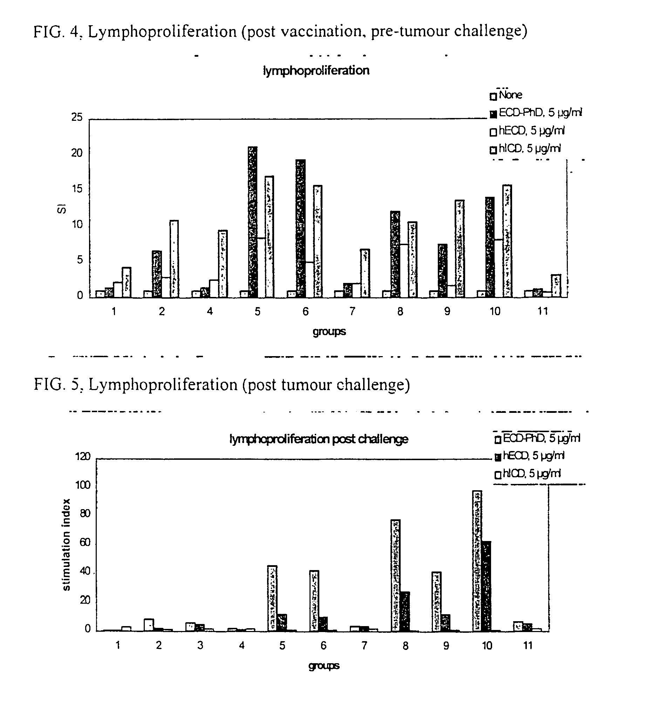

Figures 4 and 5 show the lymphoproliferation of splenocytes in vitro after incubation with the 5µg/ml of immunogen (ECD-PD) or extracellular domain (ECD) or intracellular domain (ICD) or Her2Neu.

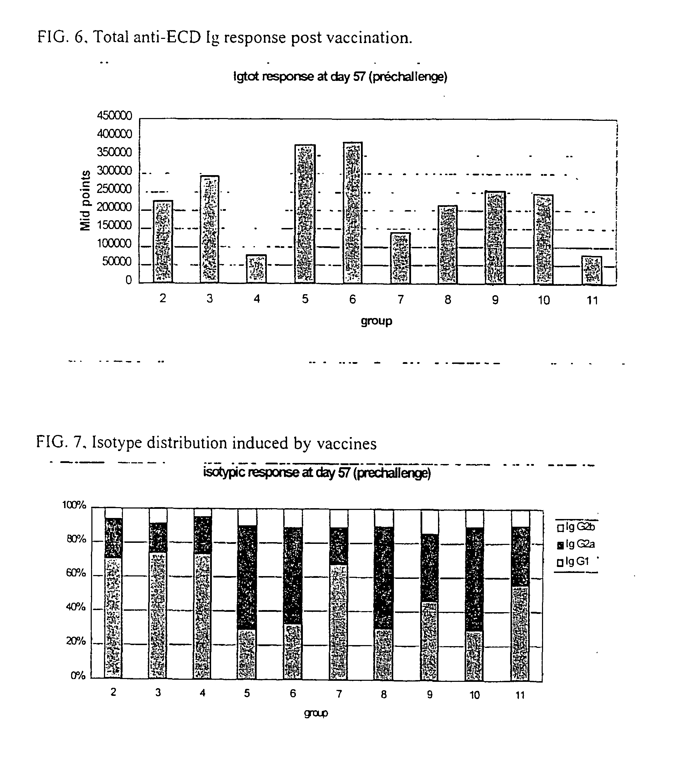

Figures 6 and 7 show the humoral immune response to the immunogen (ECD-PD) in terms of total Ig as measured by ELISA (FIG. 6) or IgG isotype distribution within these responses (FIG. 7).

### Example 2, Immunisation of mice with P703P antigen

This experiment was designed to investigate a range of adjuvant formulations with the antigen which is a fusion of the antigen Prostase (Ferguson, et al. (Proc. Natl. Acad. Sci. USA 1999, 96, 3114-3119) and the N-terminal 1-81 fragment of NS1 from the Influenza virus (P703P-NS1).

| Group | Antigen (25µg) | Adjuvant |
|---|---|---|
| 1 | P703P-NS1 | none (Phosphate Buffered Saline (PBS)) |
| 2 | P703P-NS1 | CpG |
| 3 | P703P-NS1 | Liposomes with QS21 in membrane + CpG |
| 4 | P703P-NS1 | Liposomes with QS21 and 3D-MPL in membrane + CpG |
| 5 | P703P-NS1 | tocol containing oil in water emulsion with QS21 and 3D-MPL + CpG |
| 6 | P703P-NS 1 | tocol containing oil in water emulsion + CpG |

The tocol containing oil in water emulsions used in the above groups used D. L. α-tocopherol (CAS No. 10191-41-0; chemical name: (2RS,4'RS, 8'RS)-2, 5, 7, 8-tetramethyl-2-(4', 8', 12'-trimethyl-tridecyl)-6-chromanol)); which is commercially available from ROCHE™. If present the tocol was present in an oil in water emulsion comprising 2.5% by volume, in combination with squalene 2.5% by volume. Both oils were mixed, and polyoxyethylene sorbitan monooleate (Tween 80™) was added, prior to microfluidisation (M110S microfluidics machine, maximum of 50 passes, for a period of 2 minutes at maximum pressure input of 6 bar (output pressure of about 850 bar) as described in WO 95/17210). Accordingly, groups 5 and 6 were based on the above tocol emulsion with the addition of aqueous QS21, 3D-MPL and/or CpG.

QS21 and 3D-MPL if present in any of the vaccine groups above were included at 5µg/dose; CpG (OLIGO 4 (SEQ ID NO:4): TCG TCG TTT TGT CGT TTT GTC GTT) was added at 50µg dose.

The adjuvants as used for group 3 and 4 were prepared according to techniques as described in EP 0 822 831 B1

### Vaccination procedure

Groups of B6F 1 mice were vaccinated on four occasions (in 50µl volumes), intramuscularly, 14 days apart.

### Results

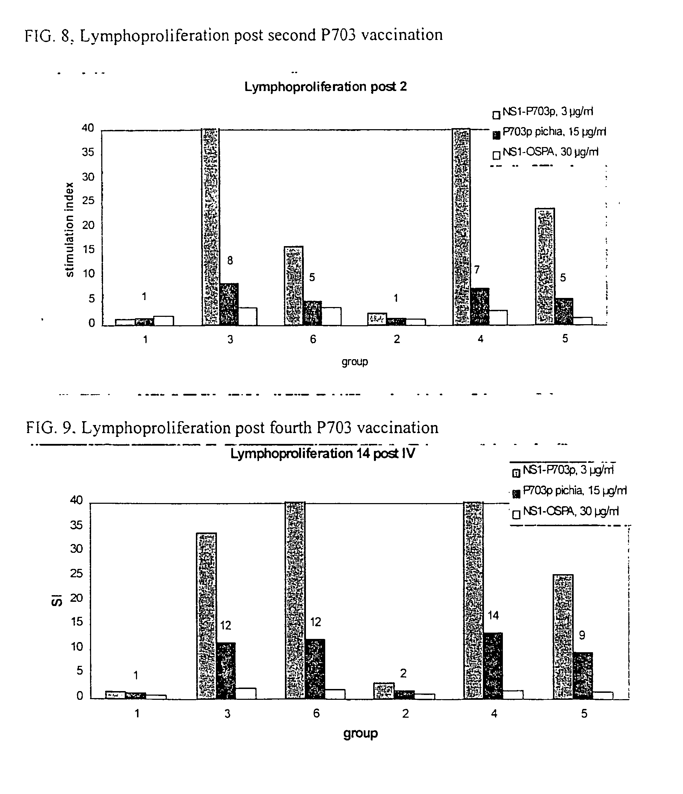

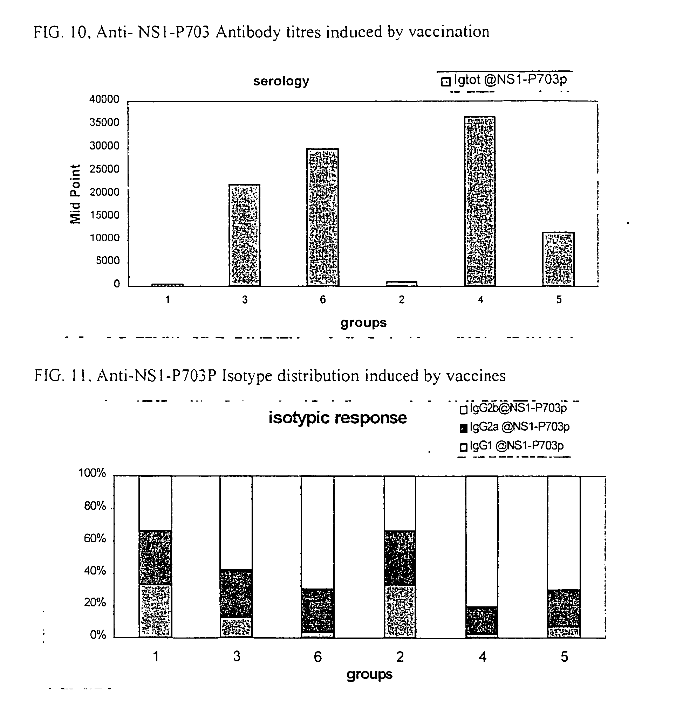

Figures 8 and 9 show the in vitro lymphoproliferation of splenocytes post second and 14 days post fourth vaccinations, after in vitro incubation with the 3µg/ml of immunogen (NS1-P703P) or pichia expressed P703P (15µg/ml) or a non-specific NS1-OspA fusion protein.

Figures 10 and 11 show the humoral immune response to the immunogen (NS1-P703P) in terms of total Ig as measured by mid-point titre ELISA (FIG. 10) or IgG isotype distribution within these responses (FIG. 11).

## Claims

1. An adjuvant composition comprising a combination of an immunostimulatory oligodeoxynucleotide and a tocol.

2. An adjuvant composition as claimed in claim 1 wherein the tocol is in the form of an oil in water emulsion.

3. An adjuvant composition as claimed in claim 2 wherein the oil in water emulsion further comprises squalene.

4. An adjuvant composition as claimed in any one of claims 1 to 3, wherein said immunostimulatory oligodeoxynucleotide comprises a Purine, Purine, C, G, pyrimidine, pyrimidine sequence, wherein the C and G are unmethylated.

5. An adjuvant composition as claimed in claims 1 to 3, wherein said immunostimulatory oligodeoxynucleotide is selected from the group comprising:
TCC ATG ACG TTC CTG ACG TT (SEQ ID NO:1); TCT CCC AGC GTG CGC CAT (SEQ ID NO:2); ACC GAT GAC GTC GCC GGT GAC GGC ACC ACG (SEQ ID NO:3); TCG TCG TTT TGT CGT TTT GTC GTT (SEQ ID NO:4); TCC ATG ACG TTC CTG ATG CT (SEQ ID NO:S).

6. An adjuvant composition according to claim 1 to 3, wherein the immunostimulatory oligodeoxynucleotide contains at least two unmethylated CG repeats being separated at least by 3 nucleotides.

7. An adjuvant composition according to claim 6, wherein the immunostimulatory oligodeoxynucleotide contains at least two unmethylated CG repeats being separated by 6 nucleotides.

8. An adjuvant composition as claimed in claim 1 wherein the tocol is described by the general formula: 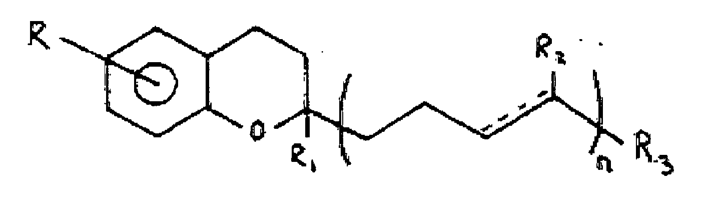 wherein R may be H or one or more identical or different substituents chosen from the group comprising alkyl, alkoxy, acyloxy, hydroxy, a sulphate and a phosphate group; R1 and R3 independently of one another are H or alkyl; R2 is H or alkyl and may be different in each unit; the broken line indicates the presence or absence of an additional carbon-carbon bond in a unit; and n= the value 1 to 10, wherein the alkyl group in R, R1, R2 and R3 may be chosen in particular from a linear or branched carbon chain having 1-4 carbon atoms , such as methyl, ethyl, butyl or isobutyL

9. An adjuvant composition as claimed in claim 8, wherein the tocol is D, L, α-tocopherol.

10. An adjuvant composition as claimed in any one of claims 1 to 9, wherein said adjuvant further comprises an additional immunostimulant.

11. An adjuvant composition as claimed in claim 10 wherein the additional immunostimulant is selected from LPS or a derivative thereof, 3D-MPL, a saponin, or QS21.

12. A vaccine composition comprising an adjuvant composition as claimed in any one of claims 1 to 11, and an antigen or antigenic composition.

13. A vaccine as claimed in claim 12, wherein the antigen is ECD-PD.

14. Use of an immunostimulatory oligodeoxynucleotide and a tocol containing oil in water emulsion in the manufacture of a medicament for shifting the quality of an immune response against an antigen towards a Th1-type immune response..

15. Use according to Claim 14 wherein the combination of the immunostimulatory oligodeoxynucleotide with a tocol containing oil in water emulsion generates a Th1-type immune response such that when antigen specific IgG isotypes induced by the vaccine after vaccination of a mouse are measured, IgG1 constitutes less than 50% of the total antigen specific IgG as determined by mid point titres measured by isotype specific ELISA.

16. A method of manufacturing a vaccine composition comprising formulating an oil in water emulsion comprising a tocol, admixing said tocol emulsion with an immunostimulatory oligodeoxynucleotide to form an adjuvant, and formulating said adjuvant with an antigen or antigenic composition.

17. A vaccine as claimed in claim 12 for use in medicine.

## Patentansprüche

1. Adjuvanszusammensetzung, die eine Kombination aus einem immunstimulierenden Oligodesoxynukleotid und einem Tocol umfaßt.

2. Adjuvanszusammensetzung gemäß Anspruch 1, worin das ' Tocol in der Form einer Öl-in-Wasser-Emulsion ist.

3. Adjuvanszusammensetzung gemäß Anspruch 2, worin die Öl-in-Wasser-Emulsion ferner Squalen umfaßt.

4. Adjuvanszusammensetzung gemäß einem der Ansprüche 1 bis 3, worin das immunstimulierende Oligodesoxynukleotid eine Sequenz Purin, Purin, C, G, Pyrimidin, Pyrimidin umfaßt, worin das C und G unmethyliert sind.

5. Adjuvanszusammensetzung gemäß den Ansprüchen 1 bis 3, worin das immunstimulierende Oligodesoxynukleotid aus der Gruppe ausgewählt ist, die folgendes umfaßt:
TCC ATG ACG TTC CTG ACG TT (SEQ ID NO: 1);
TCT CCC AGC GTG CGC CAT (SEQ ID NO: 2);
ACC GAT GAC GTC GCC GGT GAC GGC ACC ACG (SEQ ID NO: 3);
TCG TCG TTT TGT CGT TTT GTC GTT (SEQ ID NO: 4);
TCC ATG ACG TTC CTG ATG CT (SEQ ID NO: 5).

6. Adjuvanszusammensetzung gemäß den Ansprüchen 1 bis 3, worin das immunstimulierende Oligodesoxynukleotid mindestens zwei unmethylierte CG-Wiederholungen enthält, die durch mindestens 3 Nukleotide getrennt sind.

7. Adjuvanszusammensetzung gemäß Anspruch 6, worin das immunstimulierende Oligodesoxynukleotid mindestens zwei unmethylierte CG-Wiederholungen enthält, die durch 6 Nukleotide getrennt sind.

8. Adjuvanszusammensetzung gemäß Anspruch 1, worin das Tocol durch die allgemeine Formel 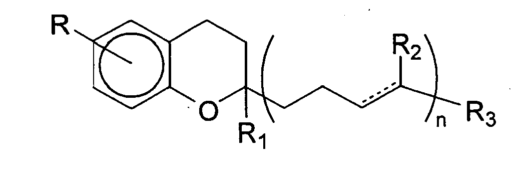 beschrieben ist, worin R H oder ein oder mehrere identische oder verschiedene Substituenten sein kann, die aus der Gruppe ausgewählt sind, die folgende umfaßt: Alkyl, Alkoxy, Acyloxy, Hydroxy, eine Sulfat- und eine Phosphatgruppe;
R₁ und R₃ unabhängig voneinander H oder Alkyl sind; R₂ H oder Alkyl ist und in jeder Einheit unterschiedlich sein kann; die gestrichelte Linie die Gegenwart oder Abwesenheit einer zusätzlichen Kohlenstoff-Kohlenstoff-Bindung in einer Einheit anzeigt; und n = der Wert 1 bis 10, worin die Alkylgruppe in R, R₁, R₂ und R₃ insbesondere aus einer geraden oder verzweigten Kohlenstoffkette mit 1 bis 4 Kohlenstoffatomen ausgewählt sein kann, wie z.B. Methyl, Ethyl, Butyl oder Isobutyl.

9. Adjuvanszusammensetzung gemäß Anspruch 8, worin das Tocol D,L-α-Tocopherol ist.

10. Adjuvanszusammensetzung gemäß einem der Ansprüche 1 bis 9, worin das Adjuvans ferner ein zusätzliches Immunstimulans umfaßt.

11. Adjuvanszusammensetzung gemäß Anspruch 10, worin das zusätzliche Immunstimulans ausgewählt ist aus LPS oder einem Derivat davon, 3D-MPL, einem Saponin oder QS21.

12. Impfstoffzusammensetzung, die eine Adjuvanszusammensetzung gemäß einem der Ansprüche 1 bis 11 und ein Antigen oder eine antigene Zusammensetzung umfaßt.

13. Impfstoff gemäß Anspruch 12, worin das Antigen ECD-PD ist.

14. Verwendung eines immunstimulierenden Oligodesoxynukleotids und einer Tocol-haltigen Öl-in-Wasser-Emulsion bei der Herstellung eines Medikaments zur Verschiebung der Qualität einer Immunantwort gegen ein Antigen in Richtung auf eine Th1-Typ-Immunantwort.

15. Verwendung gemäß Anspruch 14, worin die Kombination aus dem immunstimulierenden Oligodesoxynukleotid und einer Tocol-haltigen Öl-in-Wasser-Emulsion eine Th1-Typ-Immunantwort erzeugt, so daß, wenn Antigen-spezifische IgG-Isotypen, die durch den Impfstoff nach Impfung einer Maus induziert werden, gemessen werden, IgG1 weniger als 50 % der gesamten Antigen-spezifischen IgGs ausmacht, wie durch Isotyp-spezifischen ELISA gemessene Mittelpunkttiter bestimmt.

16. Verfahren zur Herstellung einer Impfstoffzusammensetzung, das das Formulieren einer Öl-in-Wasser-Emulsion, die ein Tocol umfaßt, das Mischen der Tocolemulsion mit einem immunstimulierenden Oligodesoxynukleotid zur Bildung eines Adjuvans und das Formulieren des Adjuvans mit einem Antigen oder einer antigenen Zusammensetzung umfaßt.

17. Impfstoff gemäß Anspruch 12 zur Verwendung in der Medizin.

## Revendications

1. Composition adjuvante comprenant une combinaison d'un oligodésoxynucléotide immunostimulateur et un tocol.

2. Composition adjuvante telle que revendiquée dans la revendication 1, dans laquelle le tocol est sous la forme d'une émulsion huile dans l'eau.

3. Composition adjuvante telle que revendiquée dans la revendication 2, dans laquelle l'émulsion huile dans l'eau comprend, en outre, un squalène.

4. Composition adjuvante telle que revendiquée dans l'une quelconque des revendications 1 à 3, dans laquelle ledit oligodésoxynucléotide immunostimulateur comprend une Purine, Purine, C, G, pyrimidine, séquence pyrimidine, où C et G sont non méthylés.

5. Composition adjuvante telle que revendiquée dans les revendications 1 à 3, dans laquelle ledit oligodésoxynucléotide immunostimulateur est choisi dans le groupe comprenant :
TCC ATG ACG TTC CTG ACG TT (SEQ ID NO:1); TCT CCC AGC GTG CGC CAT (SEQ ID NO:2);ACCGATGAC GTC GCC GGT GAC CGC ACC AGG (SEQ ID NO: 3);TCG TCG TTT TGT CGT TTT GTC GTT (SEQ ID -NO:4); TCC ATG ACG TTC CTG ATG CT (SEQ ID NO:5).

6. Composition adjuvante selon les revendications 1 à 3, dans laquelle l'oligodésoxynucléotide immunostimulateur contient au moins deux répétitions CG non méthylées séparées par au moins 3 nucléotides.

7. Composition adjuvante selon la revendication 6, dans laquelle l'oligodésoxynucléotide immunostimulateur contient au moins deux répétitions CG non méthylées séparées par au moins 6 nucléotides.

8. Composition adjuvante telle que revendiquée dans la revendication 1, dans laquelle le tocol est décrit par la formule générale : 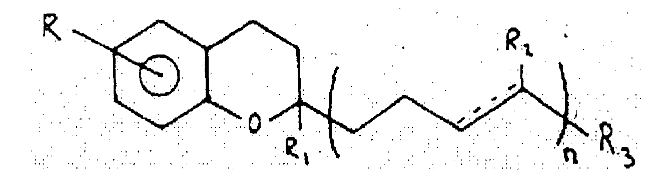 dans laquelle R peut être H ou un ou plusieurs substituants identiques ou différents choisis dans le groupe comprenant alkyle, alcoxy, acyloxy, hydroxy, un groupe sulfate et phosphate ;
R1 et R3 indépendamment l'un de l'autre sont H ou alkyle ; R2 est H ou alkyle et peut être différent dans chaque unité ; la ligne brisée indique la présence ou l'absence d'une liaison carbone-carbone supplémentaire dans une unité ; et n = la valeur de 1 à 10, dans laquelle le groupe alkyle dans R, R1, R2 et R3 peut être choisi en particulier parmi une chaîne de carbones linéaire ou ramifiée ayant de 1 à 4 atomes de carbone, telle que méthyle, éthyle, butyle ou isobutyle.

9. Composition adjuvante telle que revendiquée dans la revendication 8, dans laquelle le tocol est un D-, L-, α-tocophérol.

10. Composition adjuvante telle que revendiquée dans l'une quelconque des revendications 1 à 9, dans laquelle ledit adjuvant comprend, en outre, un immunostimulant supplémentaire.

11. Composition adjuvante telle que revendiquée dans la revendication 10, dans laquelle l'immunostimulant supplémentaire est choisi parmi les LPS, ou un dérivé de ceux-ci, 3D-MPL, une saponine, ou QS21.

12. Composition vaccinale comprenant une composition adjuvante telle que revendiquée dans l'une quelconque des revendications 1 à 11, et un antigène ou une composition antigénique.

13. Vaccin tel que revendiqué dans la revendication 12, dans lequel l'antigène est ECD-PD.

14. Utilisation d'un oligodésoxynucléotide immunostimulateur et d'une émulsion huile dans l'eau contenant un tocol dans la fabrication d'un médicament destiné à déplacer la qualité d'une réponse immunitaire dirigée contre un antigène vers une réponse immunitaire de type Th1.

15. Utilisation selon la revendication 14, dans laquelle la combinaison de l'oligodésoxynucléotide immunostimulateur avec une émulsion huile dans l'eau contenant un tocol génère une réponse immunitaire de type Th-1 de telle façon que, quand les isotypes IgG spécifiques de l'antigène induits par le vaccin après vaccination d'une souris sont mesurés, IgG1 constitue moins de 50 % des IgG spécifiques de l'antigène totales, comme déterminé par les titres au point milieu mesurés par une ELISA spécifique des isotypes.

16. Procédé de fabrication d'une composition vaccinale comprenant la formulation d'une émulsion huile dans l'eau comprenant un tocol, le mélange de ladite émulsion de tocol avec un oligodésoxynucléotide immunostimulateur pour former un adjuvant, et la formulation dudit adjuvant avec un antigène ou une composition antigénique.

17. Vaccin tel que revendiqué dans la revendication 12 utilisable en médecine.
